# EUROPEAN PATENT SPECIFICATION

(11) **EP 2 810 629 B1**
(45) Date of publication and mention of the grant of the patent: **09.11.2016**
(21) Application number: 14170460.1
(22) Date of filing: 29.05.2014
(51) Int. Cl.: A61F 13/15, A61F 13/472, A61F 13/49, A61F 13/505, A61F 13/66

(54) **Sleeve for an absorbent pad and nappy-knickers comprising said sleeve**
Umhüllung für ein absorbierendes Kissen und Höschenwindel enthaltend die Umhüllung
Enveloppe d'insertion pour une serviette absorbante ed couche comprenant ladite enveloppe d'insertion

(30) Priority: 06.06.2013 IT BS20130084
(43) Date of publication of application: 10.12.2014
(73) Proprietor: Steinmann & Co. S.a.S. Di Colombo Cristina, 20857 Camparada, Monza-Brianza (IT)
(72) Inventor: Corona Steinmann, Valentina, 20857 Camparada, MONZA-BRIANZA (IT)
(74) Representative: Chimini, Francesco

(56) References cited:
- EP-A2- 2 359 789
- WO-A2-2007/073506
- DE-A1- 4 133 456
- US-A- 3 658 064
- US-A1- 2004 158 225
- US-A1- 2012 010 585
- US-A1- 2013 023 846

## Description

The present invention relates to a sleeve for an absorbent pad suitable for being positioned in the crotch area of an adult, child or infant. The invention further relates to nappy-knickers comprising such sleeve for an absorbent pad.

Nappy-knickers are already known, both for adults and children, comprising an enclosure wherein an absorbent pad is inserted. The enclosure is in turn attached, for example by means of press-studs, to knickers. EP 2 359 789 A2 discloses a reusable diaper shaped as a pair of pants and designed to house a removable absorbent insert. US 2012/010585 A1 discloses a reusable diaper including forward and rearward waist portions. At least one corner tab may be releasably attachable to the forward and rearward waist portions. The diaper may include at least one pocket configured to receive therein at least a portion of the corner tab. Other examples diapers are known from US 2013/023846 A1, US 2004/158225 A1, US 3 658 064 A, WO 2007/073506 A2 and DE 41 33 456 A1.

In the attempt to minimize the seal against leaks of the absorbent pad, the known nappy-knickers have some disadvantages as regards comfort. For example, the sleeve for an absorbent pad does not have a shape that adapts perfectly to the anatomy of the crotch area and also the coupling with the knickers may cause some disadvantages. For example, the attachment means between the sleeve for an absorbent pad and the knickers contact the skin of the user and may leave marks on the same skin. In some embodiments, in order to ensure the seal, the sleeve has curls along the edges contacting the skin, which prevent even the smallest relative movements between the sleeve and the skin and which may also cause inconveniences and marks left on the skin.

Object of the present invention is to propose a sleeve for an absorbent pad and nappy-knickers comprising such sleeve, capable of obviating the drawbacks of the prior art diapers.

Another object of the invention is to propose a sleeve for an absorbent pad and nappy-knickers capable of providing the greatest comfort for the user and at the same time capable of ensuring an optimum seal against leaks.

A further object of the invention is to provide a sleeve for an absorbent pad and nappy-knickers which are particularly simple to manufacture and assemble.

Said objects are achieved with a sleeve for an absorbent pad according to claim 1 and with nappy-knickers according to claim 9. The dependent claims describe preferred embodiments of the invention.

The features and the advantages of the invention shall be made readily apparent in the description that follows of some preferred embodiments thereof, provided purely by way of non limiting indications, with reference to the accompanying figures, in which:
Figure 1 shows part of a material roll from which a plurality of sleeves for an absorbent pad according to the invention is obtained;
Figures 2 and 2a are schematic plan views of the sleeve in two steps of its formation;
Figure 3 is a perspective view of the formed sleeve and of an absorbent pad therein;
Figure 4 is a top plan view of the sleeve with absorbent pad;
Figure 5 is a view with separated parts of nappy-knickers according to the invention, comprising an absorbent pad, a sleeve and nappy-knickers;
Figures 6, 6a and 6b schematically show as many possible embodiments of the lateral edge of the knickers;
Figures 7 and 7a show, in front and rear view, the nappy-knickers worn by a child;
Figure 8 is a view of nappy-knickers in an embodiment variation; and
Figure 9 is a view of nappy-knickers in a further embodiment variation.

In said drawings, 1 indicates a sleeve for an absorbent pad suitable for containing an absorbent pad 100 so that, in use, the assembly of the sleeve 1 and the absorbent pad 100 housed therein may be positioned in the crotch area of an adult, child or infant. The sleeve 1 and the skin of the person enclose the absorbent pad 100 and form a barrier against any leaks in the absorbent pad.

The sleeve 1 comprises a sleeve body 3 made in a waterproof material. The sleeve body 3 forms a front pocket 5 suitable for receiving a first end of the absorbent pad 100 and a rear pocket 7 suitable for receiving a second end of the absorbent pad 100.

The sleeve body is made from a flat cloth 300 of a trapezoidal shape defined by a rear edge 301, a front edge 302, parallel to and inferior in length to said rear edge, and two oblique lateral edges 303.

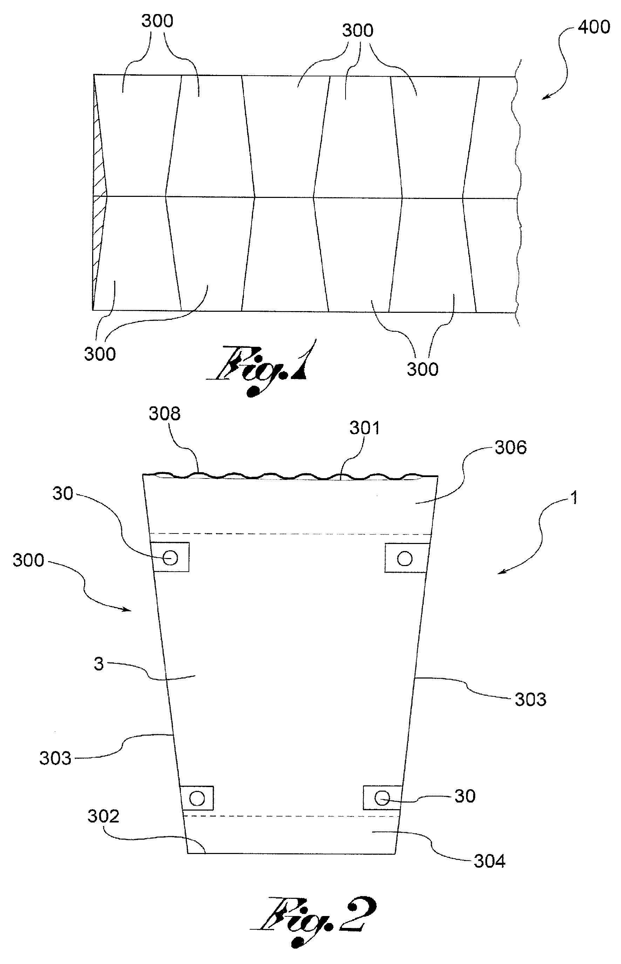

Figure 1 shows an example of a roll portion 400 of a waterproof material from which a plurality of material cloths 300 is obtained with which to form sleeves 1 for an absorbent pad. In particular, the material roll 400 is divided into two halves in the longitudinal direction and each half is cut transversally with oblique cuts so as to obtain adjacent cloths 300 without waste of material between two flanking cloths. If the roll 400 has a width which is double the distance between the rear 301 and front 302 edges of the material cloths 300, the waste is null also in the transversal direction. Otherwise, in any case a waste in the form of a rectangular strip is obtained which may be easily reused.

For example, the waterproof material making the sleeve 1 is a synthetic or a mixed synthetic and natural fabric, for example polyester-cotton blend, laminated internally, that is on the side wherein the absorbent pad rests, with a waterproof material, such as polyurethane.

Referring now to a single cloth as obtained from the waterproof material roll of Figure 1, end portions 304, 306 of said cloth ending in said front and rear edges are folded inwards and sewn to the lateral edges 303 so as to form the front pocket 5 and the rear pocket 7.

Furthermore, the rear edge 301 and the lateral edges 303 are elasticised, while the front edge 302 is not elasticised. It is not necessary for the front edge 302 to be elastic since it already has a width lower than that of the rear edge 301. The absence of any elastic in the front edge 302 increases the comfort since such edge remains softer, compresses less and does not leave marks on the belly of the user.

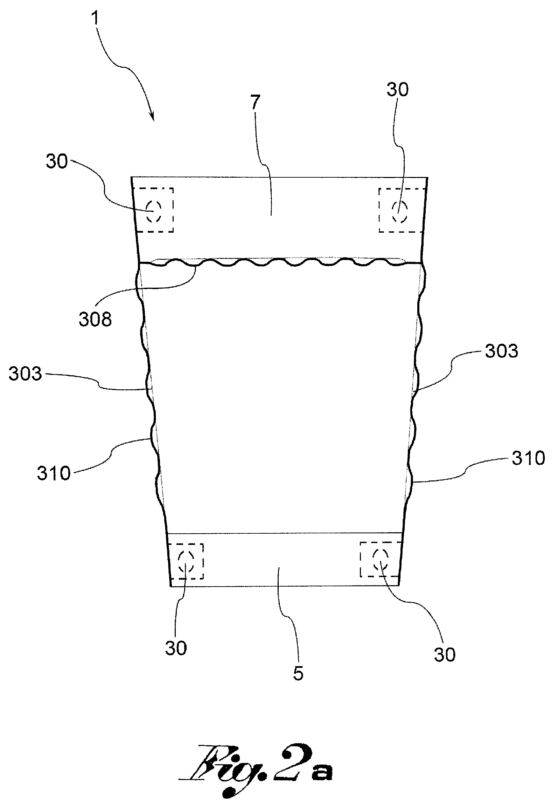

In a preferred embodiment, a rear elastic tape 308 (schematically indicated by a thicker line in Figure 2) is sewn to the rear edge 301 before the end portions 304, 306 of the cloth 300 are folded inwards and sewn to the lateral edges 303 (Figure 2). In this step, any buttons 30, which will be discussed below, are attached to the cloth 300.

After the end portions 304, 306 of the cloth have been folded inwards, side elastic tapes 310 (schematically indicated by a thicker line in Figure 2a) are sewn to the lateral edges 303, so that said lateral tapes 310 form the front 5 and rear 7 pockets (Figure 2a). In other words, the lateral elastic tapes 310 perform the double function of making the lateral edges 303 of the sleeve elastic and of sewing the front 5 and rear 7 pockets.

Preferably, as will be explained hereinafter, the lateral elastic tapes 310 are placed in tension in the intermediate portion only of the respective lateral edges 303 not forming the pockets. In other words, while the intermediate portion of the sleeve 1 is elasticised so that it adapts to the size and anatomy of the user, at the level of the front 5 and rear 7 pockets the sleeve 1 does not form curls and remains substantially flat and soft.

Advantageously, the elastic tapes 308, 310 are made in a soft fabric and in any case suitable for contacting the skin of the user. For example, said elastic tapes 308, 310 are strings made from a first non-elastic yarn, for example polyamide, combined with a second elastic yarn, for example elastan. Such tapes provide a rest surface on the skin which is smooth, soft and free from curls.

It should be noted that the parts of the sleeve contacting the skin of the user are, besides the elastic tapes 308, 310, the outer sides 5', 7' of the front 5 and rear 7 pockets which, being obtained by folding inwards the end portions 304, 306 of the starting cloth 300, coincide with the outer side of said cloth, which is not coated by the waterproof material.

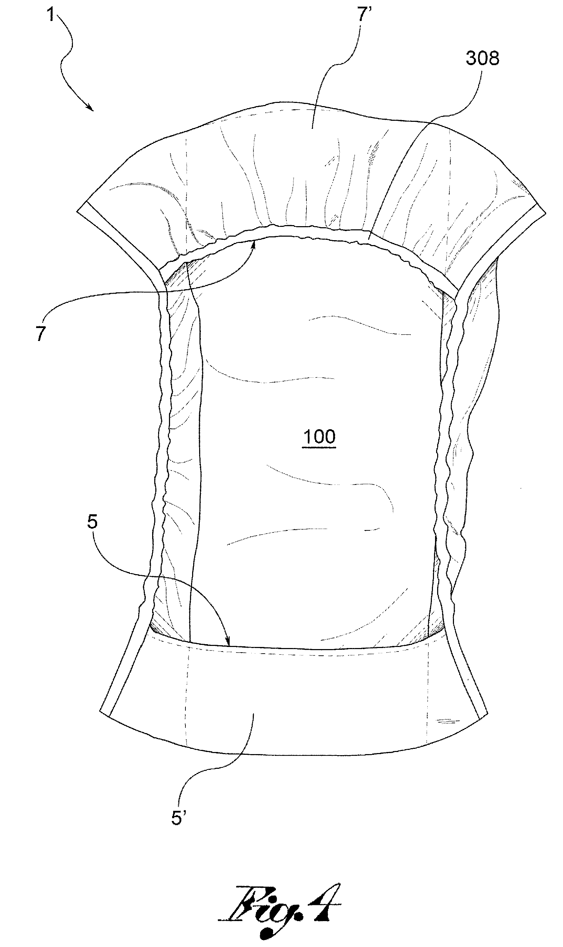

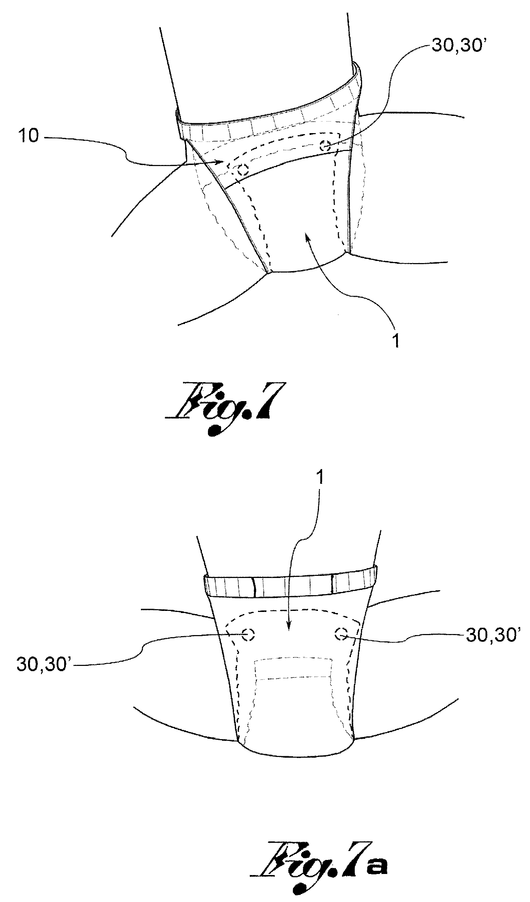

In one embodiment illustrated in Figures 2-7, the sleeve 1 is suitable for being used with knickers 10 to form an object generally known as nappy-knickers.

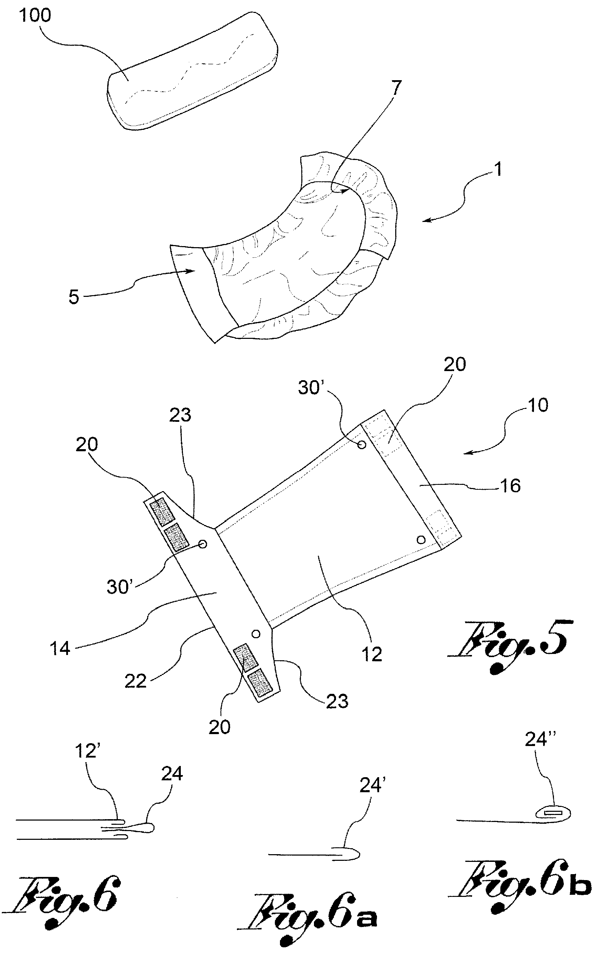

As can be appreciated in particular by the view with separated parts of Figure 5, the knickers 10 comprise an intermediate portion 12 suitable for being positioned in the crotch area of an adult, child or infant, a front portion 14 and a rear portion 16. Such front 14 and rear 16 portions are suitable for being tied together at the user's waist. For example, said front 14 and rear 16 portions of the knickers 10 are provided with reciprocal coupling means, for example, using Velcro strips 20, 21', press-studs, laces or other releasable attachment members.

The knickers 10 may be mad in a natural or mixed natural and synthetic fabric.

The sleeve 1 is predominantly positioned on the inner side of said intermediate portion 12.

In a preferred embodiment, the sleeve 1 is removably attached to the knickers. This makes washing and drying the nappy-knickers easier, for example because the sleeve laminated with a polyurethane material may not be suitable for being dried with an automatic dryer, unlike the knickers.

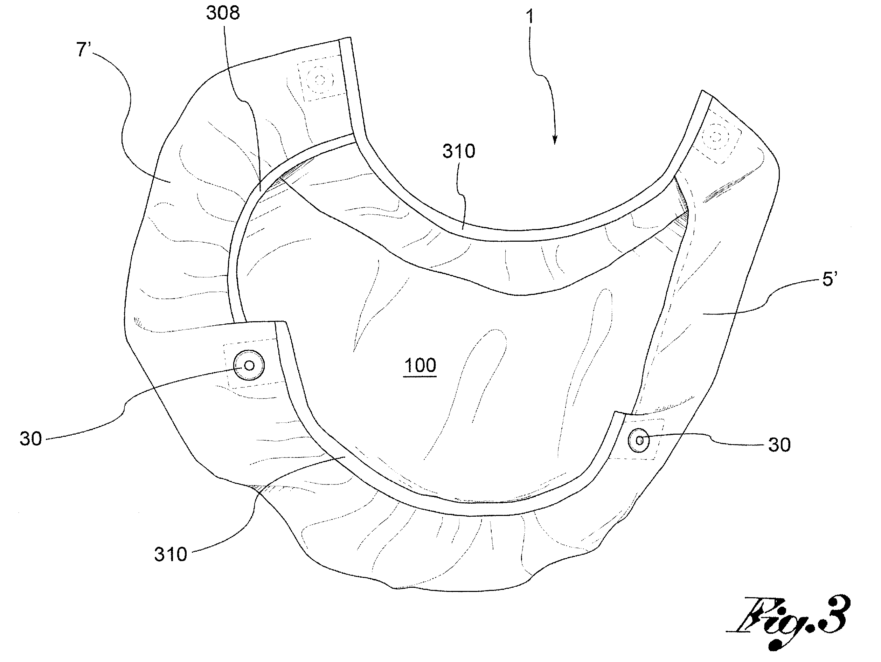

In the illustrated embodiment, the sleeve 1 is coupled to the knickers by means of press-studs 30, 30'. As can be appreciated in particular in Figures 2 and 3, four buttons 30 of the sleeve 1 are positioned in the proximity of the lateral edges 303 and at the height of the front 5 and rear 7 pockets, that is in the proximity of the corners of the sleeve when the pockets have been sewn. Such positioning of the buttons inside the pockets 5, 7, unlike the outer buttons relative to the sleeve as in the objects of the prior art, prevents the skin of the user from becoming marked by the same buttons. Moreover, the positioning in the proximity of the corners allows the sleeve 1 to be tensioned as much as possible when it is attached to the knickers 10, preventing any undesired crinkling. Yet still, the positioning of the buttons 30 under the pockets 5, 7, where the fabric is not crinkled being the elastic tape free from tension as explained hereinbefore, advantageously allows attaching the buttons 30, 30' to a flat area and therefore parallel to the inner side of the knickers 10.

In a preferred embodiment, a pair of rear buttons 30' of the knickers is positioned in the intermediate portion 12 in the proximity of the rear portion 16 and a pair of front buttons 30' is positioned in the front portion 14. In this way, the greatest extension of the sleeve 1 and therefore the complete coverage of the crotch area of the user, even on the front, are guaranteed.

In a preferred embodiment, the intermediate position of the knickers 12 has a trapezoidal shape, substantially in relation to the trapezoidal shape of the fabric cloth 300 forming the sleeve 1. As explained in relation to the sleeve 1, also for the knickers such trapezoidal shape, besides following the anatomy of the crotch area, allows minimising the scraps when cutting the intermediate portion of the knickers from a fabric roll.

In a preferred embodiment, the front portion 14 of the knickers 10 comprises an elastic band 22 having a transversal dimension greater than the width of the intermediate portion. Such elastic band 22 connects to the intermediate portion 12 by means of arched portions 23.

Furthermore, such elastic band 22 has such a height so as to be shaping as well as soft and comfortable.

In one embodiment, the knickers 10 are made with a double fabric. In this case, an elastic leg gripper 24 may be sewn between the two borders 12' forming the lateral edge of the intermediate portion 12 (Figure 6). In an embodiment variation, the intermediate portion 12 is made in a single layer of fabric and the elastic leg gripper 24' is sewn to said layer (Figure 6a). In a further embodiment variation, the intermediate portion 12 is made in a single layer of fabric which, along the lateral edges, forms a curl wherein an elastic 24" is inserted.

In an embodiment variation, the sleeve 1 is permanently attached to the knickers 10, for example by stitching.

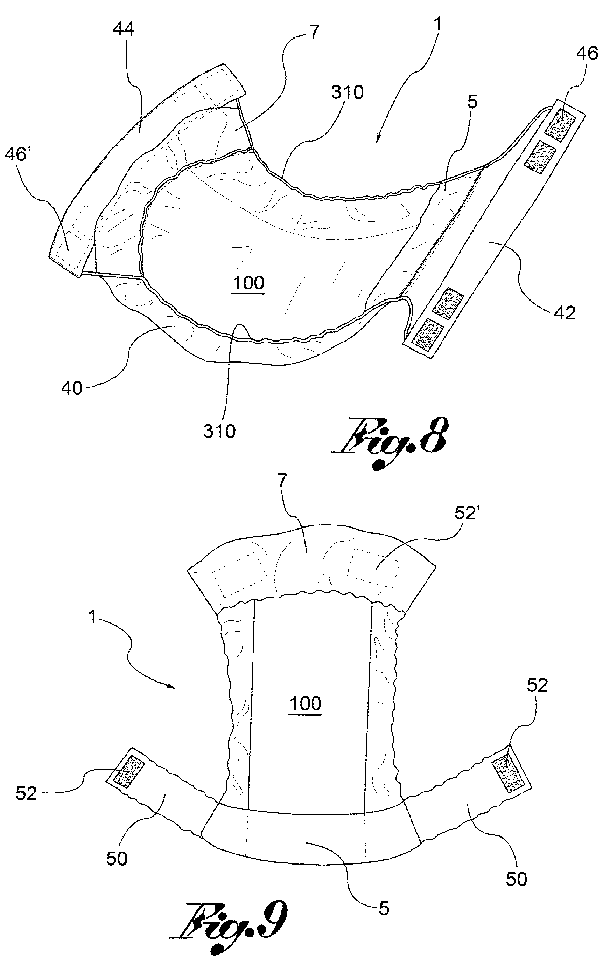

In a further embodiment illustrated in Figure 8, the sleeve 1 is not coupled to proper knickers, but is sewn to an outer covering 40 which protrudes with respect to the front pocket 5 and the rear pocket 7 so as to form a front band 42 and a rear band 44 carrying reciprocal attachment means 46, 46', for example of the Velcro type. Such front 42 and rear 44 bands are suitable for being tied together at the user's waist. Preferably, the outer covering 40 is sewn to the sleeve 1 along the lateral edges thereof, advantageously by means of the same elastic tapes 310 forming the pockets 5, 7.

In the embodiment variation of Figure 9, on the contrary, two lateral tabs 50 extend from the front pocket 5 provided with front attachment means 52 suitable for tying to corresponding rear attachment means 52' made, for example, on the outer side of the rear pocket 7. Said lateral tabs 50 allow the sleeve 1 to be worn without using knickers 10 or an outer covering 40.

It is also an object of the invention a sleeve or nappy-knickers as described hereinabove, wherein an absorbent pad 100 is inserted in the sleeve.

In one embodiment, the absorbent pad 100 has a rectangular shape. The absorbent pad 100 is simply inserted in the pockets 5, 7 of the sleeve without using attachment means. In this way, the absorbent pad separates from the sleeve 1 when machine washed, without having to extract it manually.

In an embodiment variation, the absorbent pad has a trapezoidal shape corresponding to the trapezoidal shape of the sleeve.

As regards the material of the absorbent pad, in one embodiment the absorbent pad has the outer layer facing the skin in cotton or in a wavy bamboo yarn. The opposite outer layer may be in micropile, which is very draining. On the inside there may be provided one or more layers of foam, for example of microfibre.

A man skilled in the art may make several changes, adjustments and replacements of elements with other functionally equivalent ones to the embodiments of the sleeve for an absorbent pad and the nappy-knickers according to the invention in order to meet incidental needs, without departing from the scope of the following claims. Each of the features described as belonging to a possible embodiment can be obtained independently of the other embodiments described.

## Claims

1. Sleeve for an absorbent pad (100), comprising a sleeve body (3) made in waterproof material, said sleeve body (3) forming a front pocket (5) suitable for receiving a first end of an absorbent pad (100) and a rear pocket (7) suitable for receiving a second end of the absorbent pad, opposite the first, so that in use, the assembly of the sleeve and of the absorbent pad housed in the sleeve is suitable for being positioned in the crotch area of an adult, child or infant, the sleeve and the skin of the person enclosing the absorbent pad (100) and forming a barrier against leaks of the absorbent pad, the sleeve body (3) being made from a flat cloth (300) of a trapezoidal shape defined by a rear edge (301), a front edge (302), parallel to and inferior in length to said rear edge, and two oblique lateral edges (303), wherein end portions (304, 306) of said cloth (300) ending in said front and rear edges are folded inwards and sewn to the lateral edges (303) so as to form the front pocket (5) and the rear pocket (7);
said sleeve being **characterized in that** only the rear edge (301) and the lateral edges (303) are elasticised.

2. Sleeve according to claim 1, wherein a rear elastic tape (308) is sewn onto the rear edge (301) and lateral elastic tapes (310) are sewn onto the lateral edges (303) after the end portions of the cloth (304, 306) have been folded inwards, in such a way that said lateral tapes (310) form the front (5) and rear (7) pocket.

3. Sleeve according to claim 2, wherein the lateral elastic tapes (310) are placed in tension in the intermediate portion only of the respective lateral edges not forming the pockets.

4. Sleeve according to any of the previous claims, further comprising a front band (42), which extends beyond the front pocket (5), and a rear band (44), which extends beyond the rear pocket (7).

5. Sleeve according to claim 4, wherein said front and rear bands are provided with connection means (46, 46') for a reciprocal lacing around the user's waist.

6. Sleeve according to the previous claim, wherein said front (42) and rear (44) bands are made at the ends of an outer covering (40) sewn to the sleeve.

7. Sleeve according to claims 2 and 6, wherein said outer covering (40) is sewn to the lateral edges (303) of the sleeve.

8. Sleeve according to any of the claims 1-3, wherein two lateral tabs (50) extend from the front pocket (5) provided with front attachment means (52) suitable for tying to corresponding rear attachment means (52') made, for example, on the outer side of the rear pocket (7).

9. Nappy-knickers, comprising a sleeve (1) according to any of the claims 1-3 and a knickers (10), where said knickers comprise an intermediate portion (12) a front portion (14) and a rear portion (16), suitable for being tied together at the user's waist, the sleeve being positioned mainly on the inner side of said intermediate portion (12).

10. Nappy-knickers according to the previous claim, wherein the sleeve is detachably attached to the knickers.

11. Nappy-knickers according to the previous claim, wherein the sleeve and the knickers are fitted with press-studs (30, 30') for their detachable coupling.

12. Nappy-knickers according to the previous claim, wherein the studs of the sleeve (30) are positioned at the corners and inside the front and rear pockets.

13. Nappy-knickers according to the previous claim, wherein the knickers are fitted with a pair of front buttons (30') positioned in the front part (14).

14. Nappy-knickers according to any of the claims 9-13, wherein the intermediate portion (12) of the knickers has a trapezoidal shape suited to the trapezoidal shape of the sleeve.

## Patentansprüche

1. Hülle für ein Absorbenskissen (100), die einen Hüllenkörper (3) umfasst, der aus einem wasserfesten Material hergestellt ist, wobei der Hüllenkörper (3) eine vordere Tasche (5), die zum Aufnehmen eines ersten Endes eines Absorbenskissens (100) geeignet ist, und eine hintere Tasche (7) bildet, die zum Aufnehmen eines zweiten Endes des Absorbenskissens geeignet ist und dem ersten Ende gegenüber liegt, so dass im Gebrauch die Anordnung aus der Hülle und dem Absorbenskissen, das in der Hülle aufgenommen ist, zum Positionieren in dem Schrittbereich eines Erwachsenen, eines Kindes oder Säuglings oder Kleinkinds geeignet ist, wobei die Hülle und die Haut der Person das Absorbenskissen (100) umgeben und eine Barri-ere gegen eine Leckage des Absorbenskissens bilden, wobei der Hüllenkörper (3) aus einem flachen Gewebe (300) mit einer Trapezform hergestellt ist, die durch eine hintere Kante (301), eine vordere Kante (302), die parallel zu der hinteren Kante und kürzer als diese ist, und zwei schräge Seitenkanten (303) festgelegt ist, wobei End-abschnitte (304, 306) des Gewebes (300), die in der vorderen und der hinteren Kante enden, einwärts gefaltet sind und derart an die Seitenkanten (303) genäht sind, dass sie die vordere Tasche (5) und die hintere Tasche (7) bilden,
wobei die Hülle **dadurch gekennzeichnet ist, dass** nur die hintere Kante (301) und die Seitenkanten (303) elastisch gemacht sind.

2. Hülle nach Anspruch 1, bei der ein hinteres elastisches Band (308) auf die hintere Kante (301) genäht wurde und seitliche elastische Bänder (310) an die Seitenkanten (303) genäht wurden, nachdem die Endabschnitte des Gewebes (304, 306) einwärts gefaltet worden sind, und zwar derart, dass die seitlichen Bänder (310) die vordere (5) und die hintere (7) Tasche bilden.

3. Hülle nach Anspruch 2, bei der die seitlichen elastischen Bänder (310) unter Spannung in dem Zwischenabschnitt nur der jeweiligen Seitenkanten, die nicht die Taschen bilden, angeordnet sind.

4. Hülle nach einem der vorhergehenden Ansprüche, die ferner ein vorderes Band (42), das sich über die vordere Tasche (5) hinaus erstreckt, und ein hinteres Band (44), das sich über die hintere Tasche (7) hinaus erstreckt, umfasst.

5. Hülle nach Anspruch 4, bei der das vordere und das hintere Band mit Verbindungsmitteln (46, 46') für ein wechselseitiges Schnüren um die Taille eines Anwenders ausgestattet sind.

6. Hülle nach dem vorhergehenden Anspruch, bei der das vordere (42) und das hintere (44) Band an den Enden einer äußeren Abdeckung (40) ausgebildet sind, die an die Hülle genäht ist.

7. Hülle nach Anspruch 2 und 6, bei der die äußere Abdeckung (40) an die Seitenkanten (303) der Hülle genäht ist.

8. Hülle nach einem der Ansprüche 1 bis 3, bei der sich zwei seitliche Laschen (50) von der vorderen Tasche (5) erstrecken und mit vorderen Anbringungsmitteln (52) ausgestattet sind, die zum Verbinden mit entsprechenden hinteren An-bringungsmitteln (52') geeignet sind, die beispielsweise auf der Außenseite der hinteren Tasche (7) ausgebildet sind.

9. Windelhöschen, das eine Hülle (1) nach einem der Ansprüche 1 bis 3 und ein Höschen (10) umfasst, wobei das Höschen einen Zwischenabschnitt (12), einen vorderen Abschnitt (14) und einen hinteren Abschnitt (16) umfasst, die dazu geeignet sind, an der Taille eines Anwenders miteinander verbunden zu werden, wobei die Hülle vorwiegend auf der Innenseite des Zwischenabschnitts (12) angeordnet ist.

10. Windelhöschen nach dem vorhergehenden Anspruch, bei dem die Hülle lösbar an dem Höschen angebracht ist.

11. Windelhöschen nach dem vorhergehenden Anspruch, bei dem die Hülle und das Höschen mit Druckknöpfen (30, 30') für deren lösbares Verbinden ausgestattet sind.

12. Windelhöschen nach dem vorhergehenden Anspruch, bei dem die Knöpfe der Hülle (30) an den Ecken und innerhalb der vorderen und hinteren Tasche angeordnet sind.

13. Windelhöschen nach dem vorhergehenden Anspruch, bei dem das Höschen mit einem Paar von vorderen Knöpfen (30') angebracht ist, die in dem vorderen Teil (14) angeordnet sind.

14. Windelhöschen nach einem der Ansprüche 9 bis 13, bei dem der Zwische-nabschnitt (12) des Höschens eine Trapezform aufweist, die zu der Trapezform der Hülle passt.

## Revendications

1. Enveloppe pour coussinet absorbant (100), comprenant un corps d'enveloppe (3) en matière imperméable, ledit corps d'enveloppe (3) formant une poche avant (5) adaptée à recevoir une première extrémité d'un coussinet absorbant (100) et une poche arrière (7) adaptée à recevoir une seconde extrémité du coussinet absorbant, opposée à la première, de telle sorte qu'en utilisation, l'ensemble de l'enveloppe et du coussinet absorbant logé dans l'enveloppe est adapté à être positionné dans la zone d'entrejambe d'un adulte, d'un enfant ou d'un bébé, l'enveloppe et la peau de la personne enfermant le coussinet absorbant (100) et formant une barrière contre les fuites du coussinet absorbant, le corps d'enveloppe (3) étant réalisé à partir d'un tissu plat (300) d'une forme trapézoïdale définie par un bord arrière (301), un bord avant (302), parallèle audit bord arrière et de longueur inférieure à ce dernier, et deux bords latéraux obliques (303), dans laquelle des parties d'extrémité (304, 306) dudit tissu (300) se terminant auxdits bords avant et arrière sont pliées vers l'intérieur et cousues aux bords latéraux (303) de manière à former la poche avant (5) et la poche arrière (7) ;
ladite enveloppe étant **caractérisée en ce que** seul le bord arrière (301) et les bords latéraux (303) sont élastiques.

2. Enveloppe selon la revendication 1, dans laquelle un cordon élastique arrière (308) est cousu sur le bord arrière (301) et des cordons élastiques latéraux (310) sont cousus sur les bords latéraux (303) après que les parties d'extrémité du tissu (304, 306) ont été pliées vers l'intérieur, de telle manière que lesdits cordons latéraux (310) forment la poche avant (5) et la poche arrière (7).

3. Enveloppe selon la revendication 2, dans laquelle les cordons élastiques latéraux (310) sont placés en tension uniquement dans la partie intermédiaire des bords latéraux respectifs ne formant pas les poches.

4. Enveloppe selon l'une quelconque des revendications précédentes, comprenant en outre une bande avant (42), qui s'étend au-delà de la poche avant (5), et une bande arrière (44), qui s'étend au-delà de la poche arrière (7).

5. Enveloppe selon la revendication 4, dans laquelle lesdites bandes avant et arrière sont pourvues de moyens de liaison (46, 46') pour un raccordement réciproque autour de la taille de l'utilisateur.

6. Enveloppe selon la revendication précédente, dans laquelle lesdites bandes avant (42) et arrière (44) sont réalisées aux extrémités d'une protection extérieure (40) cousue à l'enveloppe.

7. Enveloppe selon les revendications 2 et 6, dans laquelle ladite protection extérieure (40) est cousue aux bords latéraux (303) de l'enveloppe.

8. Enveloppe selon l'une quelconque des revendications 1-3, dans laquelle deux languettes latérales (50) s'étendent à partir de la poche avant (5) pourvues de moyens de fixation avant (52) adapté à s'attacher à des moyens de fixation arrière (52') correspondant réalisés, par exemple, sur le côté extérieur de la poche arrière (7).

9. Couche-culotte, comprenant une enveloppe (1) selon l'une quelconque des revendications 1-3 et une culotte (10), où ladite culotte comprend une partie intermédiaire (12), une partie avant (14) et une partie arrière (16), appropriées pour être attachées ensemble au niveau de la taille de l'utilisateur, l'enveloppe étant positionnée principalement sur le côté intérieur de ladite partie intermédiaire (12).

10. Couche-culotte selon la revendication précédente, dans laquelle l'enveloppe est fixée de manière détachable à la culotte.

11. Couche-culotte selon la revendication précédente, dans laquelle l'enveloppe et la culotte sont équipées de boutons à pression (30, 30') pour leur couplage détachable.

12. Couche-culotte selon la revendication précédente, dans laquelle les boutons de l'enveloppe (30) sont positionnés au niveau des coins et à l'intérieur des poches avant et arrière.

13. Couche-culotte selon la revendication précédente, dans laquelle la culotte est équipée d'une paire de boutons avant (30') positionnées dans la partie avant (14).

14. Couche-culotte selon l'une quelconque des revendications 9-13, dans laquelle la partie intermédiaire (12) de la culotte a une forme trapézoïdale adaptée à la forme trapézoïdale de l'enveloppe.
